Europäisches Patentamt

European Patent Office

Office européen des brevets

(19)

(11) Veröffentlichungsnummer : **0 100 037**
**B1**

(12) EUROPÄISCHE PATENTSCHRIFT

(45) Veröffentlichungstag der Patentschrift :
21.01.87

(51) Int. Cl.⁴ : **A 61 K 31/515** // (A61K31/515, 31:495)

(21) Anmeldenummer : 83106977.8

(22) Anmeldetag : 15.07.83

(54) Arzneimittel zur Behandlung von Schlafstörungen.

(30) Priorität : 29.07.82 DE 3228351

(43) Veröffentlichungstag der Anmeldung :
08.02.84 Patentblatt 84/06

(45) Bekanntmachung des Hinweises auf die Patenterteilung : 21.01.87 Patentblatt 87/04

(84) Benannte Vertragsstaaten :
AT BE CH DE FR GB IT LI LU NL SE

(56) Entgegenhaltungen :
DE-B- 1 086 235
FR-M- 4 135
ARZNEIMITTEL-FORSCHUNG, Band 25, Nr. 9, September 1975, Seiten 1408-1413, Editio Cantor KG, Aulendorf, DE; L.K.C. DESMEDT et al.: "Anticonvulsive properties of cinnarizine and flunarizine in rats and mice"

(73) Patentinhaber : **Merckle GmbH**
**Dr.-Georg-Spohn-Strasse 7**
**D-7902 Blaubeuren (DE)**

(72) Erfinder : **Metz, Gunter, Dr.**
**Auf dem Rucken 29**
**D-7902 Blaubeuren (DE)**
Erfinder : **Räuchle, Kurt, Dr.**
**Forstweg 16**
**D-7902 Blaubeuren-Sonderbuch (DE)**

(74) Vertreter : **Lederer, Franz, Dr. et al**
**Vanderwerth, Lederer & Riederer Patentanwälte Lucile-Grahn-Strasse 22**
**D-8000 München 80 (DE)**

## Beschreibung

Die Erfindung betrifft ein Arzneimittel zur Behandlung von Schlafstörungen, das in niedriger Dosierung ein oder mehrere der zu den Barbitüraten zählenden Verbindungen, ausgewählt aus der Gruppe, die aus Phenobarbital, Cyclobarbital-Calcium und/oder Hexobarbital besteht, in Kombination mit Cinnarizin und/oder Flunarizin enthält.

Unter den therapeutisch verwendeten Hypnotika besitzen die Barbiturate noch heute eine zentrale Stellung. Von Nachteil in der Anwendung ist jedoch u. a. die Notwendigkeit hoher Dosierung, die je nach Barbiturat Einzeldosen von 150 mg bis etwa 650 mg erfordert. Aus der Literatur sind verschiedene Stoffe bekannt, die Barbiturate in ihrer Wirkung verstärken können. So hemmt Dimercaprol (BAL) den Abbau von Pentobarbital [J. Pharmacol exp. Therap. *109*, 292 (1953)], auch Tocopherol verstärkt die Wirkung der Barbiturate [Arch. int. Pharmacodyn. Therap. *97*, 473 (1954)]. Daneben ist bekannt, daß starke Antihistaminika (Phenothiazine) und Tranquillizer (Meprobamat), auf Barbiturate einen potenzierenden Einfluß zu haben. Unter den gebräuchlichen Kombinationen von Barbituraten mit Sedativa und anderen Hypnotika, die in der « Rote Liste » aufgeführt sind, sind auch noch wenige Vertreter dieser beiden Gruppen enthalten.

Es wurde nun überraschend gefunden, daß Cinnarizin und Flunarizin auf Phenobarbital, Cyclobarbital-Calcium und Hexobarbital wirkungsverstärkend wirken und diese Kombinationen neue pharmakologische Eigenschaften besitzen. Cinnarizin ist aus der DE-PS 1 086 235 bekannt. Cannarizin und auch sein difluoriertes Strukturanalog Flunarizin besitzen nur schwach ausgeprägte antihistaminische Eigenschaften und werden therapeutisch als periphere und cerebrale Vasodilatatoren verwendet.

Aus der FR-M-4 135 ist ein Arzneimittel zur Behandlung von Angina pectoris bekannt, das Amobarbital und Cinnarizin enthält.

Gegenstand der Erfindung ist ein Arzneimittel, das dadurch gekennzeichnet ist, daß es neben üblichen pharmazeutischen Hilfs- und Trägerstoffen als Wirkstoff eine Kombination von

a) einer oder mehreren Verbindungen, ausgewählt aus der Gruppe bestehend aus Phenobarbital, Cyclobarbital-Calcium und/oder Hexobarbital und deren pharmazeutisch verwendbaren Salzen sowie

b) Cinnarizin oder Flunarizin bzw. ein pharmazeutisch verwendbares Salz dieser Verbindungen enthält.

Der erfindungsgemäße Arzneimittel ist zur Behandlung von Schlafstörungen hervorragend geeignet.

Zweckmäßigerweise enthält das erfindungsgemäße Arzneimittel die unter a) (Phenobarbital, Cyclobarbital-Calcium und/oder Hexobarbital) und b) (Cinnarizin oder Flunarizin) aufgeführten Komponenten in einem Gewichtsverhältnis von $a : b$ von $100 : 1$ bis $5 : 1$, vorzugsweise von $50 : 1$ bis $10 : 1$. Vorzugsweise wird das erfindungsgemäße Arzneimittel für die orale und rektale Applikation formuliert. Je Dosiseinheit enthält das erfindungsgemäße Arzneimittel zweckmäßigerweise 100-150 mg der Komponente (a) und 5-15 mg Cinnarizin ($b_1$) oder 2-5 mg Flunarizin ($b_2$).

Im allgemeinen beträgt das Gewichtsverhältnis der unter a) aufgeführten Komponenten zu Cinnarizin ($b_1$) $30 : 1$ bis $5 : 1$, vorzugsweise $20 : 1$ bis $10 : 1$, und das Gewichtsverhältnis der unter a) aufgeführten Komponenten zu Flunarizin ($b_2$) beträgt $100 : 1$ bis $20 : 1$, vorzugsweise $50 : 1$ bis $30 : 1$.

Der Einfluß von Cinnarizin und Flunarizin auf die Schlafzeitverlängerung wurde an männlichen NMRJ-Mäusen modifiziert nach « Screening Methods in Pharmacology », R.A. Turner, 1976, Seite 70, bestimmt. Es wurde Phenobarbital (Barbiturat mit langdauernder Wirkung), Cyclobarbital-Calcium (mit kurzer bis mittlerer Wirkung), sowie Hexobarbital (ultrakurze Wirkung), sowie eine Kombination zweier Barbiturate (Cyclobarbitural-Ca + Hexobarbital) in abgestuften. Dosierungen oral appliziert. Die für eine 30 minütige Schlafzeit erforderliche Dosierung wurde als $ED_{50}$ berechnet. Im weiteren Testverlauf erhielten die Tiere die jeweilige $ED_{50}$ als schlaferzeugende Grunddosis sowie Cinnarizin oder Flunarizin in abgestuften Dosierungen. Für die Kombination wurde die $ED_{50}$ mittels Probit Analyse berechnet, wobei die jeweilige Dosierung von Cinnarizin oder Flunarizin verwendet wurde, bei der die Ausgangsschlafzeit um 50 % erhöht wurde. Die Ergebnisse dieser Versuche sind in Tabelle 1 und 2 dargestellt.

In einem erweiterten Versuch wurde der Einfluß von Cinnarizin auf die Barbiturate anhand der Meßparameter Schlafdauer, Schlafzeitverlängerung, $ED_{50}$, sowie Wirkungseintritt und Wirkungsoptimum gestestet. Die Ergebnisse dieses Versuches sind in Tabelle 3 zusammengefaßt.

Als Kriterien für den Wirkungseintritt wurde der Beginn des Exitationsstadiums gewertet. Für das Wirkungsoptimum wurde der Beginn der Schlafphase alle 10 Minuten der Schwanz- und Cornealreflex (nach Irvin) nach folgendem Schema überprüft :

0 = normale Reaktion
— 1 = leicht vermindert
— 2 = deutlich vermindert
— 3 = hochgradig vermindert } Wirkungsoptimum
— n = völliges Fehlen von Reaktion

Die Ergebnisse der Schlafdauer und Schlafzeitverlängerung in Tabelle 3 bestätigen die Resultate der vorhergehenden Untersuchungen (Tabelle 2). Ein entsprechender synergistischer Effekt ist sowohl bei Wirkungseintritt, wie auch Wirkungsoptimum festzustellen. Offensichtlich ist auch ein gruppenspezifischer Barbiturateffekt vorhanden. Im Falle von Hexobarbital zeigt Cinnarizin die höchste Potenz, beim Cyclobarbital-Calcium hingegen Flunarizin.

Die Kombination beider Barbiturate erfordert sowohl für Cinnarizin als auch Flunarizin höhere Dosierungen zur Erzielung einer $ED_{50}$ im Vergleich zu den Einzelbarbituraten. Der Wirkungseintritt im Vergleich zur Kontrolle wird bei der Kombination nicht verändert, jedoch bei den Einzelbarbituraten. Hier ist die ausgeprägteste Wirkung beim Cyclobarbital-Calcium festzustellen.

Aus den Ergebnissen der Tabellen 1 bis 3 geht hervor, daß ein Gemisch von Cinnarizin oder Flunarizin mit Barbituraten eine ausgeprägte Schlafzeitverlängerung verursacht, woraus zur Erzielung gleicher Wirkung erheblich geringere Dosierungen der Barbiturate möglich sind, sowie auch eine wesentliche Verbesserung des Wirkungseintrittes und des Wirkungsoptimums.

Die erfindungsgemäßen Arzneimittel werden rektal in Form von Suppositorien und Rektalkapseln und oral in Form von Tabletten, Kapseln oder Dragées verabreicht unter Verwendung üblicher pharmazeutischer Hilfsstoffe, sowie gebräuchlicher Träger-, Gleit- und Sprengmittel.

Beispiele

1. Hartgelatinekapseln

| | |
|---|---|
| Cyclobarbital-Ca | 100,0 mg |
| Flunarizin | 5,0 mg |
| Laktose D20 | 80,0 mg |
| Magnesiumstearat | 2,0 mg |
| Talkum | 8,0 mg |
| Kollidon VA64 | 5,0 mg |
| Maisstärke | 30,0 mg |

2. Tabletten

| | |
|---|---|
| Hexobarbital | 50,0 mg |
| Cyclobarbital-Ca | 100,0 mg |
| Cinnarizin | 15,0 mg |
| Hydroxypropylcellulose | 15,0 mg |
| mikrokristalline Cellulose | 103,0 mg |
| Gelatine | 5,5 mg |
| Stearinsäure | 1,5 mg |
| Talkum | 5,0 mg |

3. Suppositorien

| | |
|---|---|
| Hexobarbital | 150,0 mg |
| Cinnarizin | 10,0 mg |
| Suppositorienmasse H15 | 626,0 mg |
| Suppositorienmasse W35 | 1 264,0 mg |

(Siehe Tabellen Seite 4 ff.)

## Tabelle 1

### Bestimmung der oralen $ED_{50}$ der Barbiturate an je 10 männlichen NMRJ-Mäusen/Gruppe

| Barbiturat | Dosis mg/kg | Schlafzeit Min. | $ED_{50}$ mg/kg |
|---|---|---|---|
| Phenobarbital | 200 | 4,29 | 256 |
|  | 250 | 27,52 |  |
|  | 300 | 49,06 |  |
|  | 400 | 49,28 |  |
| Hexobarbital | 250 | 3,03 | 710 |
|  | 500 | 16,52 |  |
|  | 750 | 32,53 |  |
|  | 1000 | 56,08 |  |
| Cyclobarbital-Ca | 100 | – | 239 |
|  | 200 | 14,01 |  |
|  | 250 | 34,38 |  |
|  | 300 | $90^{1)}$ |  |
| Cyclobarbital-Ca + Hexobarbital (40 + 30) | 150 | 8,64 | 225 |
|  | 200 | 21,05 |  |
|  | 250 | 41,09 |  |
|  | 400 | $>90,0^{2)}$ |  |

[1]) 8 Tiere zeigten 90 Min., 2 Tiere 60,5 bzw. 53,3 Min.

[2]) Zwei Tiere hatten eine Schlafzeit von 67,60 bzw. 66,90 min., die restlichen > 90 min.

Tabelle 2

Bestimmung der Schlafzeitverlängerung unter dem Einfluß von Cinnarizin und Flunarizin ($ED_{50}$) 10 männliche NMRJ-Mäuse/Gruppe.

| Barbiturat | Dosis mg/kg | Cinnarizin | | | Flunarizin | | |
|---|---|---|---|---|---|---|---|
| | | Dosis mg/kg | Schlafzeit Min. | $ED_{50}$ mg/kg | Dosis mg/kg | Schlafzeit Min. | $ED_{50}$ mg/kg |
| Phenobarbital | 256 | − | 22,34 | | | | |
| | 256 | 1 | 25,78 | 3,57 | | | |
| | 256 | 5 | 32,85· | | | | |
| | 256 | 10 | 43,55* | | | | |
| Hexobarbital | 710 | − | 32,74 | | − | 38,78 | |
| | 710 | 0,5 | 35,37 | 0,75 | 1,0 | 40,44 | 2,94 |
| | 710 | 0,75 | 43,94** | | 2,5 | 45,17 | |
| | 710 | 1,0 | 65,58** | | 5,0 | 94,51*** | |
| Cyclobarbital-Ca | 239 | − | 27,45 | | − | 32,03 | |
| | 239 | 1,0 | 24,34 | 2,77 | 0,25 | 39,54 | 0,38 |
| | 239 | 2,5 | 41,43* | | 1,0 | 62,67*** | (0,29–0,45) |
| | 239 | 5,0 | 49,99** | | 2,5 | 75,15*** | |
| Cyclobarbital-Ca + Hexobarbital (40 + 30) | 225 | − | 34,27 | | − | 33,07 | |
| | 225 | 1,0 | 31,09 | 4,68 | 2,5 | 33,45 | 7,52 |
| | 225 | 2,5 | 37,92 | | 10,0 | 56,49** | (6,85–8,30) |
| | 225 | 5,0 | 53,22* | | 15,0 | 65,12*** | |

* $p < 0,05$
** $p < 0,01$
*** $p < 0,001$

0 100 037

## Tabelle 3

Schlafzeit, Wirkungseintritt und Wirkungsoptimum unter dem Einfluß von Cinnarizin an 10 männlichen NMRJ-Mäusen/Gruppe.

| Barbiturat | Dosis mg/kg | Cinnarizin mg/kg | Schlafdauer $\bar{x}$ (min.) | % | $ED_{50}$ mg/kg [2] | Wirkungseintritt $\bar{x}$ (min.) | p.a. % | min. | Wirkungsoptimum Reflexe | % 1) 2) |
|---|---|---|---|---|---|---|---|---|---|---|
| Hexobarbital | 710 | – (K) | 35,86 | 100,0 | | 2,89 | 100,0 | 10–20 | –1 | 100,0 |
| | 710 | 0,50 | 40,66 | 113,4 | 0,77 | 2,49 | 86,2 | 10–30 | –3 | 278,2–336,0 |
| | 710 | 0,75 | 42,38 | 118,2 | | 2,22 | 76,8 | 10–20 | –3 | 263,2–203,0 |
| | 710 | 1,00 | 76,14 | 212,3 · | | 1,59 | 55,0 | 10–30 | –4 | 300,8–407,0 |
| Cyclobarbital | 239 | – (K) | 24,64 | 100,0 | | 4,27 | 100,0 | 10 | 0/–1 | 100,0 |
| Calcium | 239 | 1,0 | 26,21 | 106,4 | 3,52 | 2,19 | 51,3 | 10 | –1 | 166,7 |
| | 239 | 2,5 | 31,08 | 126,1 | (3,07–4,21) | 2,31 | 54,1 | 10–30 | –1 | 350,0 |
| | 239 | 5,0 | 42,49 | 172,4 | | 2,54 | 59,5 | 10 | –2 | 350,0 |
| Cyclobarb.–Ca | 225 | – (K) | 35,01 | 100,0 | | 1,15 | 100,0 | 10–20 | –1/–2 | 100,0 |
| + Hexobarbital | 225 | 1,0 | 32,42 | 92,6 | 4,94 | 1,09 | 94,8 | 10–20 | –2 | 156,3–183,3 |
| (40 + 30) | 225 | 2,5 | 38,65 | 110,4 | (4,19–6,49) | 1,18 | 102,6 | 10–20 | –2 | 138,8–177,5 |
| | 225 | 5,0 | 54,82 | 156,6 | | 1,18 | 102,6 | 10–40 | –2/–3 | 175,0–233,0 |

1) % Werte bezogen auf den jeweiligen Barbituratwert ohne Cinnarizin (K)

2) bei starken intraindividuellen Schwankungen ist der gesamte Meßbereich (min./max. Vertrauensbereich) angegeben

**Patentansprüche** (für die Vertragsstaaten : BE, CH, DE, FR, GB, IT, LI, LU, NL, SE)

1. Arzneimittel zur Behandlung von Schlafstörungen, dadurch gekennzeichnet, daß es neben üblichen pharmazeutischen Hilfs- und Trägerstoffen als Wirkstoff eine Kombination von
  a) einer oder mehreren Verbindungen, ausgewählt aus der Gruppe bestehend aus Phenobarbital, Cyclobarbital-Calcium und/oder Hexobarbital und deren pharmazeutisch verwendbaren Salzen sowie
  b) Cinnarizin oder Flunarizin bzw. ein pharmazeutisch verwendbares Salz dieser Verbindungen enthält.

2. Arzneimittel nach Anspruch 1, dadurch gekennzeichnet, daß es die Komponente(n) der unter a) aufgeführten Verbindung(en) und b) (Cinnarizin oder Flunarizin) in einem Gewichtsverhältnis von a : b bon 100 : 1 bis 5 : 1, vorzugsweise von 50 : 1 bis 10 : 1, enthält.

3. Arzneimittel nach Anspruch 2, dadurch gekennzeichnet, daß es eine oder mehrere der unter a) aufgeführten Verbindungen oder deren pharmazeutisch verwendbare Salze und Cinnarizin oder ein pharmazeutisch verwendbares Salz davon ($b_1$) in einem Gewichtsverhältnis von a : $b_1$ von 30 : 1 bis 5 : 1, vorzugsweise von 20 : 1 bis 10 : 1, enthält.

4. Arzneimittel nach Anspruch 2, dadurch gekennzeichnet, daß es eine oder mehrere der unter a) aufgeführten Verbindungen oder deren pharmazeutisch verwendbare Salze und Flunarizin oder ein pharmazeutisch verwendbares Salz davon ($b_2$) in einem Gewichtsverhältnis von a : $b_2$ von 100 : 1 bis 20 : 1, vorzugsweise von 50 : 1 bis 30 : 1, enthält.

5. Arzneimittel nach Anspruch 3, dadurch gekennzeichnet, daß es eine Kombination von Hexobarbital und Cinnarizin enthält.

6. Arzneimittel nach Anspruch 4, dadurch gekennzeichnet, daß es eine Kombination von Cyclobarbital-Calcium und Flunarizin enthält.

7. Arzneimittel nach einem der vorgehenden Ansprüche, dadurch gekennzeichnet, daß es je Dosiseinheit 100-150 mg der unter a) aufgeführten Komponente enthält.

**Patentansprüche** (für den Vertragsstaat AT)

1. Verfahren zur Herstellung eines Arzneimittels zur Behandlung von Schlafstörungen, dadurch gekennzeichnet, daß man eine synergistisch wirksame Kombination von
  a) einer oder mehreren Verbindungen, ausgewählt aus der Gruppe bestehend aus Phenobarbital, Cyclobarbital-Calcium und/oder Hexobarbital und deren pharmazeutisch verwendbaren Salzen sowie
  b) Cinnarizin oder Flunarizin bzw. ein pharmazeutisch verwendbares Salz dieser Verbindungen hergestellt und mit üblichen pharmazeutischen Hilfs- und Trägerstoffen vermischt.

2. Verfahren nach Anspruch 1, dadurch gekennzeichnet, daß die Komponente(n) der unter a) aufgeführten Verbindung(en) und b) (Cinnarizin oder Flunarizin) in einem Gewichtsverhältnis von a : b von 100 : 1 bis 5 : 1, vorzugsweise von 50 : 1 bis 10 : 1, eingesetzt werden.

3. Verfahren nach Anspruch 2, dadurch gekennzeichnet, daß eine oder mehrere der unter a) aufgeführten Verbindungen oder deren pharmazeutisch verwendbare Salze und Cinnarizin oder ein pharmazeutisch verwendbares Salz davon ($b_1$) in einem Gewichtsverhältnis von a : $b_1$ von 30 : 1 bis 5 : 1, vorzugsweise von 20 : 1 bis 10 : 1, eingesetzt werden.

4. Verfahren nach Anspruch 2, dadurch gekennzeichnet, daß eine oder mehrere der unter a) aufgeführten Verbindungen oder deren pharmazeutisch verwendbare Salze und Flunarizin oder ein pharmazeutisch verwendbares Salz davon ($b_2$) in einem Gewichtsverhältnis von a : $b_2$ von 100 : 1 bis 20 : 1, vorzugsweise von 50 : 1 bis 30 : 1, eingesetzt werden.

5. Verfahren nach Anspruch 3, dadurch gekennzeichnet, daß eine Kombination von Hexobarbital und Cinnarizin eingesetzt wird.

6. Verfahren nach Anspruch 4, dadurch gekennzeichnet, daß eine Kombination von Cyclobarbital-Calcium und Flunarizin eingesetzt wird.

7. Verfahren nach einem der vorgehenden Ansprüche, dadurch gekennzeichnet, daß je Dosiseinheit 100-150 mg der unter a) aufgeführten Komponente verarbeitet werden.

**Claims** (for the contracting states : BE, CH, DE, FR, GB, IT, LI, LU, NL, SE)

1. Drug for the treatment of disturbed sleep, characterized in that in addition of the usual pharmaceutical auxiliary and carrier substances it contains as active ingredient a combination of
  a) one or several compounds selected from the group of phenobarbital, cyclobarbital-calcium and/or hexobarbital and their pharmaceutically usable salts as well as
  b) cinnarizine or flunarizine and/or a pharmaceutically usable salt of these compounds.

2. Drug according to claim 1, characterized in that it contains the component(s) of the compound(s) cited under a) and b) (cinnarizine or flunarizine) at a weight ratio of a : b from 100 : 1 to 5 : 1, preferably from 50 : 1 to 10 : 1.

3. Drug according to claim 2, characterized in that it contains one or several of the compounds cited under a) or their pharmaceutically usable salts and cinnarizine or a pharmaceutically usable salt thereof ($b_1$) at a weight ratio of a : $b_1$ from 30 : 1 to 5 : 1, preferably from 20 : 1 to 10 : 1.

4. Drug according to claim 2, characterized in that it contains one or several of the compounds cited under a) or their pharmaceutically usable salts and flunarizine or a pharmaceutically usable salt thereof ($b_2$) at a weight ratio of a : $b_2$ from 100 : 1 to 20 : 1, preferably from 50 : 1 to 30 : 1.

5. Drug according to claim 3, characterized in that it contains a combination of hexobarbital and cinnarizine.

6. Drug according to claim 4, characterized in that it contains a combination of cyclobarbital-calcium and flunarizine.

7. Drug according to one of the previous claims, characterized in that it contains 100-150 mg of the component cited under a) for each dosage unit.


**Claims** (for the contracting state AT)

1. Process for the production of a drug for the treatment of disturbed sleep, characterized in that a synergistically active combination of

a) one or several compounds selected from the group of phenobarbital, cyclobarbital-calcium and/or hexobarbital and their pharmaceutically usable salts as well as

b) cinnarizine or flunarizine and/or a pharmaceutically usable salt of these compounds

are produced and mixed with usual, pharmaceutical auxiliary and carrier substances.

2. Process according to claim 1, characterized in that the component(s) of the compound(s) cited under a) and b) (cinnarizine and flunarizine) are used at a weight ratio of a : b from 100 : 1 to 5 : 1, preferably from 50 : 1 to 10 : 1.

3. Process according to claim 2, characterized in that one or several of the compounds cited under a) or their pharmaceutically usable salts and cinnarizine or a pharmaceutically usable salt thereof ($b_1$) are used at a weight ratio of a : $b_1$ from 30 : 1 to 5 : 1, preferably from 20 : 1 to 10 : 1.

4. Process according to claim 2, characterized in that one or several of the compounds cited under a) or their pharmaceutically usable salts and flunarizine or a pharmaceutically usable salt thereof ($b_2$) are used at a weight ratio of a : $b_2$ from 100 : 1 to 20 : 1, preferably from 50 : 1 to 30 : 1.

5. Process according to claim 3, characterized in that a combination of hexobarbital and cinnarizine is used.

6. Process according to claim 4, characterized in that a combination of cyclobarbital-calcium and flunarizine is used.

7. Process according to one of the previous claims, characterized in that 100-150 mg of the component cited under a) is processed for each dosage unit.


**Revendications** (pour les Etats contractants : BE, CH, DE, FR, GB, IT, LI, LU, NL, SE)

1. Médicament pour le traitement de troubles du sommeil, caractérisé en ce qu'en dehors d'adjuvants et excipients pharmaceutiques usuels, il contient, en tant que substance active, une combinaison

a) d'un ou de plusieurs composés choisis dans le groupe constitué par le phénobarbital, le cyclobarbital-calcium et/ou l'hexobarbital ou leurs sels utilisables pharmaceutiquement, ainsi que

b) de cinnarizine, de flunarizine ou d'un sel utilisable pharmaceutiquement de ces composés.

2. Médicament selon la revendication 1, caractérisé en ce que ses composants, à savoir le ou les composés cités en a) et b) (cinnarizine ou flunarizine) y sont contenus dans un rapport en poids de a à b de 100 : 1 à 5 : 1, de préférence de 50 : 1 à 10 : 1.

3. Médicament selon la revendication 2, caractérisé en ce qu'il contient un ou plusieurs des composés cités en a) ou un sel utilisable pharmaceutiquement de ceux-ci et de la cinnarizine ou un sel utilisable pharmaceutiquement de celle-ci ($b_1$) dans un rapport en poids de a à $b_1$ de 30 : 1 à 5 : 1, de préférence de 20 : 1 à 10 : 1.

4. Médicament selon la revendication 2, caractérisé en ce qu'il contient un ou plusieurs des composés cités en a) ou leurs sels utilisables pharmaceutiquement et de la flunarizine ou un sel utilisable pharmaceutiquement de celle-ci ($b_2$) dans un rapport en poids de a à $b_2$ de 100 : 1 à 20 : 1, de préférence de 50 : 1 à 30 : 1.

5. Médicament selon la revendication 3, caractérisé en ce qu'il contient une combinaison d'hexobarbital et de cinnarizine.

6. Médicament selon la revendication 4, caractérisé en ce qu'il contient une combinaison de cyclobarbital-calcium et de flunarizine.

7. Médicament selon l'une quelconque des revendications 1 à 6, caractérisé en ce qu'il contient, par unité posologique, 100 à 150 mg des composants cités en a).

**Revendications** (pour l'Etat contractant AT)

1. Procédé pour la préparation d'un médicament pour le traitement de troubles du sommeil, caractérisé en ce qu'on prépare une combinaison à action synergique :

a) d'un ou de plusieurs composés choisis dans le groupe constitué par le phénobarbital, le cyclobarbital-calcium et/ou l'hexobarbital et leurs sels utilisables pharmaceutiquement, ainsi que

b) de cinnarizine ou de flunarizine ou d'un sel utilisable pharmaceutiquement de ces composés et on la mélange avec des adjuvants et des excipients pharmaceutiques usuels.

2. Procédé selon la revendication 1, caractérisé en ce que les composants, à savoir le ou les composés cités en a) et en b) (cinnarizine ou flunarizine) sont utilisés dans un rapport en poids de a à b de 100 : 1 à 5 : 1, de préférence de 50 : 1 à 10 : 1.

3. Procédé selon la revendication 2, caractérisé en ce qu'un ou plusieurs des composés cités en a) ou leurs sels utilisables pharmaceutiquement et de la cinnarizine ou un sel utilisable pharmaceutiquement de celle-ci ($b_1$) sont utilisés dans un rapport en poids de a à $b_1$ de 30 : 1 à 5 : 1, de préférence de 20 : 1 à 10 : 1.

4. Procédé selon la revendication 2, caractérisé en ce qu'un ou plusieurs des composés cités en a) ou leurs sels utilisables pharmaceutiquement et de la flunarizine ou un sel utilisable pharmaceutiquement de celle-ci ($b_2$) sont utilisés dans un rapport en poids de a à $b_2$ de 100 : 1 à 20 : 1, de préférence de 50 : 1 à 30 : 1.

5. Procédé selon la revendication 3, caractérisé en ce qu'une combinaison d'hexobarbital et de cinnarizine est utilisée.

6. Procédé selon la revendication 4, caractérisé en ce qu'une combinaison de cyclobarbital-calcium et de flunarizine est utilisée.

7. Procédé selon l'une quelconque des revendications 1 à 6, caractérisé en ce que 100 à 150 mg des composants cités en a) sont utilisés par unité posologique.